# EUROPEAN PATENT APPLICATION

(11) **EP 0 930 052 A1**
(43) Date of publication of application: **21.07.1999**
(21) Application number: 98100592.9
(22) Date of filing: 15.01.1998
(51) Int. Cl.: A61F 13/15, A61F 15/00

(54) **Package for stacked, adhesively attachable disposable absorbent articles**

(71) Applicant: THE PROCTER & GAMBLE COMPANY, Cincinnati, Ohio 45202 (US)
(72) Inventor: Hirsch, Uwe Thomas Michael Horst, 64347 Griesheim (DE)
(74) Representative: Hirsch, Uwe Thomas

(57) **Abstract**

The present invention relates to packaging for stacks of disposable absorbent articles such as sanitary napkins, pantiliners, or sweat inserts. Such articles are adhesively attached with their garment facing surface to the garment worn by the user of such articles. Stacks of disposable absorbent articles are provided by using the wearer facing surface of one article in the stack as the release/protection surface to which the adhesive on the garment facing surface of a following article is attached prior to use. In particular the present invention relates to packages for such stacks of articles wherein the package provides the release surface for the final article of a stack.

## Description

### Field of the invention

The present invention relates to packaging for stacks of disposable absorbent articles such as sanitary napkins, pantiliners, or sweat inserts. Such articles are adhesively attached with their garment facing surface to the garment worn by the user of such articles. Stacks of disposable absorbent articles are provided by using the wearer facing surface of one article in the stack as the release/protection surface to which the adhesive on the garment facing surface of a following article is attached prior to use. In particular the present invention relates to packages for such stacks of articles wherein the package provides the release surface for the final article of a stack.

### Background of the invention

Stacks of adhesively joined articles are well-known. For example the "Post It" ™ stickers by the Minnesota Mining and Manufacturing Company of Minnesota are adhesively attached to each other. They are forming a stack of stickers having a first side and a second side with the second side having an adhesive on it. The stacks are formed by attaching the adhesive of a second side of one sticker to the first surface of a following sticker within the stack. In this context the stack formation does not only provide protection of the adhesive prior to its intended use but also allows the formation of a stable stack which can be handled by the user of such stickers in a convenient fashion. The adhesive on the last sticker in each stack is protected by a disposable release liner and the stacks are packaged into a transparent film wrapper.

For disposable absorbent articles a similar approach has been disclosed in EP-A-422 922 in which an individual bandage dispenser in disclosed which comprises a continuous band of bandages which are attached with one adhesively covered side to the reverse side of the following article. These articles are, however, for application to the skin of a wearer, i.e. the adhesive is on that side which is intended to contact the ultimate user of such articles. In addition this disclosure does not disclose stacks according to the present invention since the major part of the adhesive area is still covered with release liner which in the context of band-aids also serves the function of protection of that area intended to contact the wound. The purpose of the attachment of one article to the next is to automatically transport a next article into a dispensing position when pulling an article out of the dispenser.

The disclosure of EP-A-700 675 intends to reduce the amount of release liner employed for disposable absorbent article such as sanitary napkins or pantiliners in accordance with the present invention. However, the reduction is achieved by using one release liner on both sides such that two disposable absorbent articles share one release liner. Also no stacks are formed in this context since the stacks according to the present invention have all articles directed with their garment facing surface or their wearer facing surface in the same direction. An additional problem associated with this disclosure is that the wearer facing surface of each article is exposed and that it will be contacted by the user of such articles when the first of the two articles is detached from the release liner.

WO 96/33683 discloses sanitary napkins for direct attachment to the skin of a user. In this context also stacks of such articles are considered where the wearer facing surface, which is liquid impermeable and typically provided by a liquid impermeable film functions as the release surface for the following article. However, no packaging for such articles is disclosed.

Commonly used sanitary napkins are provided with an adhesive on their garment facing surface in order to allow attachment of the disposable absorbent article to a garment of the wearer. In order to maintain this adhesive intact it must not be exposed to the environment prior to use of the absorbent article such that it is common to cover the adhesive with a protective release liner most frequently a release paper which is siliconised at least on one surface. By placing such articles on top of each other and eliminating the protective release liner stacks of absorbent articles are formed with a significant reduction in the quantity of release liner material.

It is an objective according to the present invention to provide such stacks of absorbent articles with a packaging such that the last or final article in each stack is provided with a release surface which is part of the packaging material. This satisfies several desires. Amongst others it eliminates the need to dispose of the release liner of the final article. It also provides a reduction in raw material consumption which is generally considered an environmental benefit whilst of course also reducing raw material cost of the article as delivered. Separately, a reduction in process steps, i.e. a simplification for the production of disposable absorbent articles, is achieved with the present invention while it is still possible to use commonly known process steps. Importantly by joining the stack of articles to the inside of the packaging the stacks are provided with additional stability which improves removal of the individual articles from the packaging.

Further benefits and objectives of the present invention will become more apparent when considered in the context of the drawings and the following description.

### Summary of the invention

The present invention relates to package for a stack of disposable absorbent articles each having a garment facing surface and a wearer facing surface. On the garment facing surface is an adhesive which is protected prior to use by being releasably joined to the wearer facing surface of the following article in the stack. The stacks are preferably build from at least 3, more preferably from at least 5, and most preferably from at least 10 articles stacked on top of each other. Typical articles according to the present invention are sanitary napkins, pantiliners or sweat inserts such as underarm sweat pads or shirt collar inserts. In particular pantiliners can benefit from the present invention since they are intended to be used daily or at an even higher frequency.

In order to protect the adhesive on the garment facing surface prior to use the adhesive of the last article in a stack (when considering consumption of the articles) is covered by a release surface. This release surface is comprised on the inside of the package according to the present invention. In a preferred embodiment the stack of articles is placed in a package such as a card board box wherein the surface of the package opposite the last article in the stack is providing the required protection and release function for the adhesive. Thereby no release liner is required per stack resulting in total elimination of release liner material per stack.

In a preferred embodiment the whole inside surface of the package is provided as a release surface. The release surface can be provided by coating that surface of the package material which will become the inside surface of the package. The coating can be provided for example by silicone or surfactant coating onto a polymeric or cellulose substrate.

The package according to the present invention preferably comprises an access opening to allow removal of the articles where the opening is preferably created prior to the initial use of the articles by permanently detaching a portion of the material forming the package. This can be facilitated by providing a weakening line in the package which is a well-known method well-known in particular for card board packages. Most preferably the package is a rectangular card board package created from a card board blank which prior to formation of the package is treated on one surface to provide the release surface according to the present invention inside the package. The blank is also provided with a micro perforation allowing to detach the portion forming the access opening for removal of articles from the package later on.

It is also preferable that the package contains at least two or more stacks per package and the stacks are preferably consisting of at least 5, more preferably at least 10 articles.

### Brief description of the drawings

While the specification concludes with claims particularly pointing out and distinctly claiming the present invention it is believed that the invention will be better understood from the following description in conjunction with the accompanying drawings.
Figure 1 is a perspective view of a stack of pantiliners without the package according to the present invention.
Figure 2 is a perspective view of a stack of pantiliners in a dispensing package according to the present invention.

### Detailed description of the invention

The present invention relates to packaging for disposable absorbent articles which are put above each other into a stack. The disposable absorbent articles are such articles which have an adhesive on the surface which in use is pointing away from the wearer of the article and is attached by the adhesive during use to a garment. This surface is called garment facing surface of the article while the opposite side facing the wearer during use is called wearer facing surface of the article. The adhesive on the garment facing surface of the article is a pressure sensitive adhesive which attaches to a surface upon touching it. Therefore, the adhesive requires to be protected prior to use, i.e. while the article is not yet placed on and attached to a garment.

Conventionally this protection is provided by a release liner which is a separate strip of material covering at least the adhesive region so as to prevent premature adhesion to surfaces not desired to be adhered to. According to the present invention this protection is provided by the wearer facing surface of another article such that the articles are put into a stack, one on top each other and thereby do not require the conventional release liner.

In order to provide the protection function as well as the required easy delimination the wearer facing surface of the articles have to provide the same function as a release liner while at the same time providing the usual functions of a wearer facing surface of an absorbent article such as liquid permeability. Essentially all conventional disposable absorbent articles having a wearer facing surface and a garment facing surface comprising an adhesive for attachment to a garment can benefit from the present invention. However, particularly preferred articles are sanitary napkins or pantiliners but other articles such as underarm sweat pads, shirt collar inserts or head bands can also benefit by being providing according to the present invention. The disposable absorbent articles are preferably thin, e.g. between 1 and 5 mm thick and can either be substantially flat prior to use or in a preshaped form.

The terms "joined" or "affixed", as used herein, encompasses configurations whereby a first member is directly connected to a second member and configurations whereby a first member is indirectly connected to a second member by connecting the first member to intermediate members which in turn are connected to the second member.

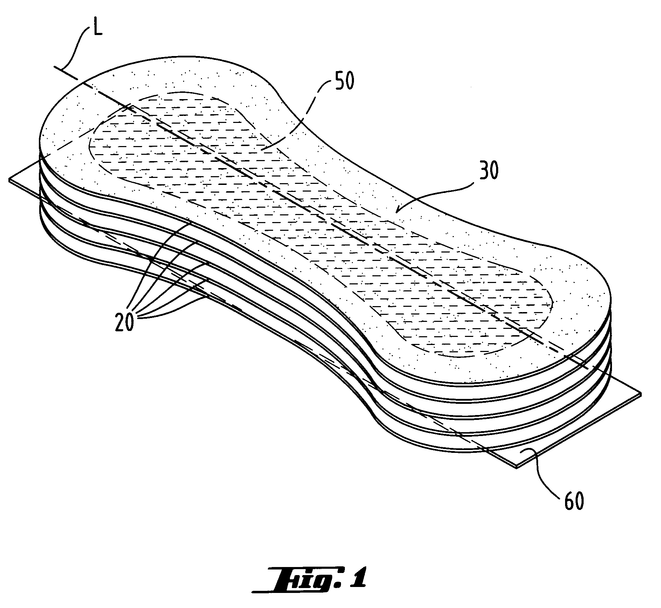

According to the present invention first a stack of articles is provided in which the adhesive on the garment facing surface of the article is joined to the wearer facing surface of the next article in order to form a stack. Referring to figure 1 a series of pantiliners, designated 20 can be seen. They are shown in a perspective view with the topsheet surface 30 visible. A panty fastening adhesive 50 is provided on the garment facing surface of the pantiliners 20. The adhesive 50 is coated on the garment facing surface within the area indicated by a dash line.

The adhesive of the upper four panty liners is protected by the wearer facing surface of the lower most four panty liners 20 while the panty fastening adhesive on the garment facing surface of the lower most pantiliner 20 in figure 1 is protected by a single release paper 60 extending beyond the longitudinal length of the pantiliners. This release paper is necessary for stacks not using the present invention. According to the present invention this release paper is replaced by (at least part of) the inside surface of the packaging. The inside surface is e.g. provided with a release coating. The pantiliners in figure 1 are stacked in registry to each other such that none extends in longitudinal direction or perpendicular thereto beyond any of the other pantiliners. The longitudinal direction is parallel to the longitudinal axis L indicated in figure 1 for the upper most pantiliner 20. As can be seen in figure 1 the panty fastening adhesive 50 does not extend to the periphery of the pantiliners 20 and in fact follows the peripheral outline of the pantiliners 20 at a fixed distance.

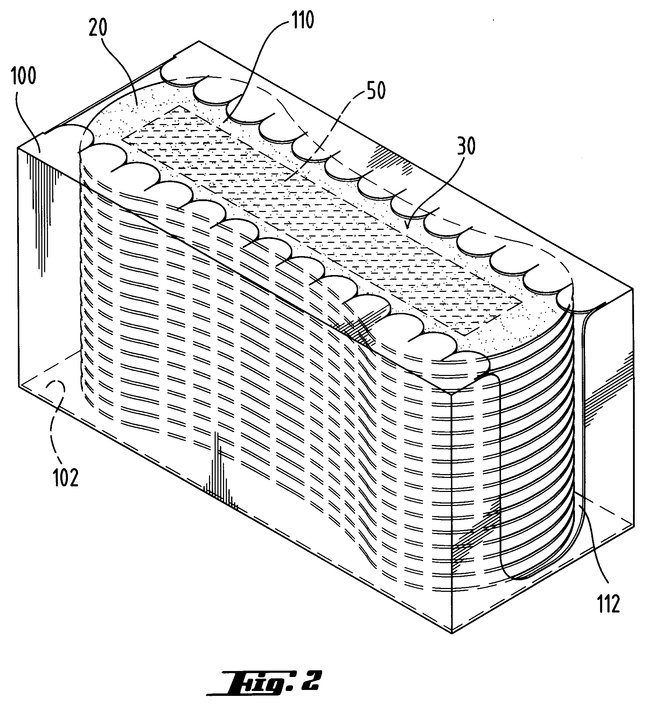

In figure 2 a similar stack, however, of more pantiliners 20 is shown in a carton package 100 according to the present invention. The panty fastening adhesive 50 again is shown on the garment facing surface indicated by dashed hatching. The wearer facing surface 30 of the pantiliners 20 is visible in the opening 110 on top of the package. In order to allow easy access to the pantiliners 20 another opening 112 is provided on one side of the package such that a wearer who wants to use the pantiliner from the stack shown in figure 2 can easily grab the upper pantiliner and delaminate it from the wearer facing surface of the underlying pantiliner.

The carton package 100 is made of such material that its bottom 102 provides a release surface for the lower most pantiliner 20 in the stack shown in figure 2 and thereby eliminates the need for any release paper for this stack. This can be provided by coating a release liner onto the bottom area 102 of the carton box at least in the area where the panty fastening adhesive of the lower most panty liner 20 will contact this part of the carton. Alternatively the whole inside surface of the carton can be coated, when e.g. the carton is formed from a blank which can easily be coated on one side, namely the side which ultimately forms the inside of the package.

The opening (110) can be provided in such a way that when removing the upper most article of a stack inside the package the side edges of the opening prevent the remainder of the stack from being pulled out. This dispensing feature helps to separate the upper most article from the remainder of the stack. This is also supported by the stack being attached through its lower most surface to the release surface inside the package. In this way both features cooperate to synergistically improve dispensing of individual articles from the packaging according to the present invention.

In a preferred embodiment the packaging according to the present invention is provided from a carton blank. The carton blank is provided with a release surface coating on the side which ultimately provides the inside of the package. This can be for example a silicon or teflon coating or a surfactant coating. The blank is then cut such that it can be folded into the packaging carton according to the present invention in such a way that the top or the bottom remains open in order to introduce the stack of articles according to the present invention. Alternatively the stack can actually be formed by guiding individual article into the package and forming the stack inside the package prior to its final closure. A perforation, micro perforation or micro slit for breaking out the openings through which the articles are intended to be dispensed when the package is in use will usually be created while the carton blank is still in a flat state.

While particular embodiments of the present invention have been illustrated and described those skilled in the art will recognize that various changes and modifications can be made. It is intended to cover in the appended claims all such modifications that are part of the present invention.

## Claims

1. A package for a stack of disposable absorbent articles, each article having a garment facing surface, a wearer facing surface and a garment facing adhesive on said garment facing surface, and said stack being formed by one article being releasable joined by said adhesive to said wearer facing surface of the following article in order to protect said adhesive prior to use, said adhesive on said garment facing surface forming one end of said stack being covered by a release surface, said package being characterised in that said package comprises on its inside said release surface.

2. A package according to claim 1 wherein the whole inside surface of said package is a release surface.

3. A package according to any of the preceding claims wherein said release surface is provided by a coating on said inside surface of said package, preferably a silicone or surfactant coating.

4. A package according to any of the preceding claims wherein said package comprise an access opening to allow removal of said articles, preferably said opening being created prior to initial use of said articles by permanently detaching a portion of the material forming said package.

5. A package according to claim 4 comprising a retarding means to maintain said stack of articles within said package while an article is delaminated from said stack and removed through said opening from said package.

6. A package according to any of the preceding claims wherein said package is rectangular, preferably said package is substantially a cardboard box.

7. A package according to any of the preceding claims wherein said package contains a quantities of 2 or more stacks per package.

8. A package according to any of the preceding claims wherein said stack comprises at least 5 articles, preferably at least 10 articles.

9. A package according to any of the preceding claims wherein said articles are sanitary napkins or panty liners.
